# EUROPEAN PATENT APPLICATION

(11) **EP 4 485 469 A1**
(43) Date of publication of application: **01.01.2025**
(21) Application number: 23182251.1
(22) Date of filing: 29.06.2023
(51) Int. Cl.: G16H 10/20, G16H 15/00, G16H 40/63, G16H 40/20, G16H 80/00, G06Q 10/06, G06Q 50/00

(54) **ELECTRONIC MEDICAL REPORT GENERATION**

(71) Applicant: Koninklijke Philips N.V., 5656 AG Eindhoven (NL)
(72) Inventor: VAN EE, Raymond, Eindhoven (NL); CHAKRABARTI, Biswaroop, Eindhoven (NL); MENA BENITO, Maria Estrella, 5656AG Eindhoven (NL); WESTERINK, Joanne Henriëtte Desirée Monique, Eindhoven (NL); RUTJES, Heleen, Eindhoven (NL); JELFS, Sam Martin, Eindhoven (NL); MENDOZA, Jose Luis Diaz, Eindhoven (NL); ANAND, Shreya, Eindhoven (NL)
(74) Representative: Philips Intellectual Property & Standards

(57) **Abstract**

Disclosed herein is a medical system (100, 600) comprising: a memory (110) storing machine executable instructions (120), receiver specific metadata (122, 122'), and database of questions (124). The receiver specific metadata comprises pointers to at least a portion of the database of questions. The medical system further comprises a computational system (104). Execution of the machine executable instructions causes the computational system to: receive (200) subject metadata (130) descriptive of a subject (618); receive (202) a selection (126) of a primary receiver; select (204) one or more questions (128) from the database of questions using the receiver specific metadata (122') of the primary receiver and the subject metadata; receive (206) sender text (132) in response to sequentially presenting the one or more questions using a sender user interface; compile (208) the sender text into an electronic medical report (134); and send (210) the electronic medical report to the primary receiver via a network connection.

## Description

### TECHNICAL FIELD

The invention relates to medical communications, in particular to the generation of electronic medical reports.

### BACKGROUND

In a standard clinical workflow, there is usually no direct communication between clinical staff members: i.e., the referring physician, the radiologist, the neuropsychologist, etc. Interdisciplinary communication in the form of medical reports (e.g., pathology reports of radiology imaging) is standard.

### SUMMARY

The invention provides for a medical system, a computer program, and a method in the independent claims. Embodiments are given in the dependent claims.

In one aspect the invention provides for a medical system that comprises a memory storing machine-executable instructions. The memory further stores receiver-specific metadata. The medical system further comprises a database of questions. The receiver-specific metadata comprises pointers to at least a portion of the database of questions.

The medical system further comprises a computational system. Execution of the machine-executable instructions causes the computational system to receive subject metadata descriptive of a subject. Execution of the machine-executable instructions further causes the computational system to receive a selection of a primary receiver. Execution of the machine-executable instructions further causes the computational system to select one or more questions from the database of questions using the receiver-specific metadata of the primary receiver and the subject metadata. Execution of the machine-executable instructions further causes the computational system to receive sender text in response to sequentially presenting the one or more questions using a sender user interface.

Execution of the machine-executable instructions further causes the computational system to compile the electronic medical report from the sender text into an electronic medical report. In this example, the various sender text in response to the sequentially presented one or more questions may be combined to form the electronic medical report. Execution of the machine-executable instructions further causes the computational system to send the electronic medical report to the primary receiver via a network connection.

In another aspect, the invention provides for a method of generating an electronic medical report. The method comprises receiving subject metadata descriptive of a subject. The method further comprises receiving a selection of a primary receiver. The method further comprises selecting one or more questions from the database of questions using the receiver-specific metadata of the primary receiver and the subject metadata. The method further comprises receiving sender text in response to sequentially presenting the one or more questions using the sender user interface. The method further comprises compiling the electronic medical report from the sender text into an electronic medical report. The method further comprises sending the electronic medical report to the primary receiver via a network connection.

In another aspect, the invention provides for a computer program that comprises machine-executable instructions. Execution of the machine-executable instructions causes a computational system to receive the subject metadata descriptive of a subject. Execution of the machine-executable instructions further causes the computational system to receive a selection of a primary receiver. Execution of the machine-executable instructions further causes the computational system to select one or more questions from a database of questions using a receiver-specific metadata of a primary receiver and the subject metadata. Execution of the machine-executable instructions further causes the computational system to receive sender text in response to sequentially presenting the one or more questions using a sender user interface. Execution of the machine-executable instructions further causes the computational system to compile the electronic medical report from the sender text into an electronic medical report. Execution of the machine-executable instructions further causes the computational system to send the electronic medical report to the primary receiver via a network connection.

### BRIEF DESCRIPTION OF THE DRAWINGS

In the following preferred embodiments of the invention will be described, by way of example only, and with reference to the drawings in which:
Fig. 1 illustrates an example of a medical system.
Fig. 2 shows a flow chart which illustrates a method of using the medical system of Fig. 1.
Fig. 3 illustrates an example of a response checking large language module.
Fig. 4 illustrates an example of a text composition large language model.
Fig. 5 illustrates an example of a sender user interface.
Fig. 6 illustrates a further example of a medical system.

### DESCRIPTION OF EMBODIMENTS

Like numbered elements in these Figures are either equivalent elements or perform the same function. Elements which have been discussed previously will not necessarily be discussed in later Figures if the function is equivalent.

A disadvantage of the current clinical communication workflow is that the reports are often not sufficiently complete or do not provide satisfactory/clear information to the receiver (e.g., not fully qualifying the indication for a study, or not precisely specifying a wanted study, or not providing relevant clinical context, or not addressing relevant clinical questions).

The advantages of examples may be that the electronic medical report is automatically constructed and is configured in a form which is beneficial or chosen by the primary receiver. The receiver-specific metadata from the primary receiver is used as well as the subject metadata, so in this way ensures that information is not missing from the electronic medical report.

In a standard clinical workflow, there is usually no direct communication between clinical staff members. Interdisciplinary communication in the form of medical reports is standard. A disadvantage of the current clinical communication workflow is that the reports are often not sufficiently complete or do not provide satisfactory/clear information to the receiver. Examples may provide a personalized adaptive module dynamically presented in the context of a specific report that is personalized and adapted to the recipient.

In some examples, the electronic medical report is sent to the primary receiver. This for example may involve the transmission to a particular physician or clinical location.

In some examples, the receiver-specific metadata may specifically identify particular questions within the database of questions. In some examples the receiver-specific metadata is metadata which is descriptive of the preferences for a particular individual or location, such as a clinic, for data or information to be contained within an electronic medical report.

The subject metadata may for example contain data, which is descriptive of a particular subject and/or information about a clinical outcome or diagnosis for a particular subject in some examples.

The primary receiver may in some examples be the intended recipient of an electronic medical report which is compiled or constructed. The primary receiver may in some examples be identification of a particular physician. In other examples the primary receiver may be identification of a particular location or organization such as a clinic or a department within a clinic or hospital.

The questions which are selected from the database of questions are therefore tailored to the particular primary receiver and the particular subject.

In different examples the sender user interface may take different forms. In some cases the sender user interface may be configured to receive text input. For example, the sender user interface could be on a mobile computing device such as a smartphone, a computer, or tablet computer which presents the one or more questions and then the user receives a text response. In other examples the sender user interface may present the information beyond just using text. For example, the sender user interface may provide text and then receive audio which is then converted into the sender text. In other examples, the sender user interface may convert the questions into an audio file, which is rendered and then played back for the primary receiver using the sender user interface. Likewise, the sender receiver text may be able to receive an audio dictation from the primary receiver using the sender user interface.

There are a variety of ways in which the electronic medical report can be compiled or constructed. In some examples, the text may be sequentially concatenated together. In other examples, there may be more complicated means, which are used to form a more readable electronic medical report such as using a large language module to combine the various portions of sender text that are received in response to sequentially presenting the one or more questions using the sender user interface.

In another example the memory further comprises a response checking module. The response checking module is configured to output an answer rating in response to receiving the one or more questions and the sender text as input. Execution of the machine-executable instructions further causes the computational system to receive the answer rating in response to inputting the one or more questions and the sender text is input. Execution of the machine-executable instructions further causes the computational system to present a sender message using the sender user interface if the answer does not meet a predetermined criterion. In this example the content of the sender text is checked against the one or more questions to see if the sender text has answered the one or more questions. This may be useful as it may provide a means for the operator of the medical system to correct the sender text before the electronic medical report is compiled. For example, the sender message may provide the opportunity to edit or alter the sender text.

In another example the response checking module is implemented using a text template. The use of a text template may be beneficial because it is possible to specify the components or parts of the text which should be within the sender text.

In another example the response checking module is implemented as a response checking large language model. The use of a large language model may be beneficial in this case because large language models are able to statistically compare text content.

A large language model (LLM) as used herein encompasses a neural network that is a language model that has been trained using unlabeled text using self-supervised or semi-supervised learning. Typically, LLMs are trained using billions of words. LLMs may for example be trained in an autoregressive form where given a segment of text the model predicts the next word (token) or words (tokens). Another mode of training is where words or tokens within a sentence are missing and the LLM predicts the missing words or tokens. Both types of LLMs may be configured to be used in the so-called prompting paradigm where a text query or statement is input into the LLM and the LLM outputs a completion or statement. The LLMs described herein are configured to operate in the prompting paradigm. Example LLMs are GPT-2, GPT-3, BERT, LLaMA, and others. The LLM may be trained for specific tasks using reinforcement learning or by reinforcement learning from human feedback (RLHF). The output of a preexisting LLM may be adjusted using fine-tuning. In fine-tuning a new set of weights connecting the final layer of the language model may be trained with specific data. Typically, this is done by freezing the other weights (other than the final output layer) in the neural network so that only the final output and format is affected.

The response checking large language model may be implemented, for example using GPT-2, GPT-3, BERT, or LLaMA. The response checking large language model may be further trained by using fine-tuning as described above to provide the answer rating. Data such as example answers with ground truth answer rating or completion text may be used for the fine-tuning process.

In another example the sender message comprises an auto complete suggestion. The addition of an auto complete suggestion may be beneficial because it may provide a means for the operator of the medical system to more rapidly correct the electronic medical report. The auto complete suggestion could for example be provided by the response checking large language model. In addition to detecting if there is content missing it may also suggest text which could be easily edited by the user or operator of the medical system.

In another example the sender message comprises an incomplete text warning. In this example, the incomplete text warning simply warns when there may be content missing from the sender text.

In another example the sender message comprises a prompt to enter additional text. This may be beneficial because it may provide a means for the operator of the medical system to add additional text and/or to edit the sender text before the electronic medical report is compiled together.

In another example, execution of the machine-executable instructions further causes the computational system to store the sender text and the sender message in the memory. Execution of the machine-executable instructions further causes the computational system to trigger the presentation of the sender message using the sender user interface if a predetermined sender activity criterion is met or after a predetermined delay. In this example, the sender text and sender message are not displayed immediately. They may for example be delayed until which time the user of the medical system, such as a physician, has time to respond to the sender message.

In another example, execution of the machine-executable instructions further causes the computational system to receive a selection of an alternate receiver. Execution of the machine-executable instructions further causes the computational system to filter and/or reformat the sender text using the receiver-specific metadata of the alternate receiver. The electronic medical report is compiled from the filtered and/or reformatted sender text. The alternate receiver may for example have a different selection of questions, which are desired to be received. In this example the sender text is reformatted or filtered such that the alternate receiver does not receive information which is not desired. This may for example enable more rapid reading or understanding of the electronic medical report for the alternate receiver.

In another example, execution of the machine-executable instructions comprises inserting medical image data descriptive of the subject into the electronic medical report. At least a portion of the subject metadata is descriptive of the medical image data. This example may be beneficial because it may provide for an improved means of presenting radiologic or other anatomical data within an electronic medical report.

In another example at least a portion of the subject text is inserted into the electronic medical report as an annotation of the medical image data. For example, portions of the subject text which are used to make the electronic medical report may be referenced when a particular portion of the medical image data is hovered over or examined by the receiver of the electronic medical report.

In another example the medical system further comprises a medical imaging system that is configured for acquiring medical imaging data descriptive of the subject. The memory further stores configuration commands configured for controlling the medical imaging system to acquire the medical imaging data. Execution of the machine-executable instructions further causes the computational system to control the medical imaging system with the configuration commands to acquire the medical imaging data from the subject. The one or more questions are least partially selected from the database of questions using the configuration commands. In this example, the configuration of the medical imaging system is used to automatically select relevant questions from the database questions relating to the protocol that was executed on the medical imaging system. This may be beneficial because it ensures that the medical image report is tailored to the information that was acquired using the electronic medical report medical imaging system.

In another example execution of the machine-executable instructions further causes the computational system to receive feedback data from the primary receiver in response to sending the electronic medical report to the primary receiver via a network connection. In one example, the feedback data is used to modify the receiver-specific metadata and/or the database questions using the feedback data. For example, the feedback data may contain a query or identification of missing or desired information which was not present in the electronic medical report. In other examples, the feedback data may be used to control or trigger pop-up messages on the sender user interface between subsequent reception of the sender text by the sender user interface. For example, this may contain hints or specific prompts for specific information when the sender text is being generated again. This may help to improve the quality of the electronic medical report.

In another example the feedback data comprises a communication score ranking the electronic medical report. This for example, could be a score assigned automatically or by the reader of the electronic medical report ranking how effective or useful the electronic medical report was. This feedback data may be used to improve the quality of the electronic medical report in the future.

In another example the feedback data comprises at least one request for clarification. The at least one request for clarification may for example be used to modify the receiver specific metadata and/or the database of questions.

In another example the feedback data comprises a number of requests for clarification. In this case, the numbers of requests for clarification are counted and the actual number of these requests is used as a rating score to determine how effective the electronic medical report was.

In another example the feedback data comprises a measured time to study the electronic medical report. This may be a useful metric to determine how quickly the electronic medical report was able to be interpreted. If the measured time is above a predetermined threshold then it may indicate that it may be beneficial to reduce the amount of information or content in future electronic medical reports.

In another example the electronic medical report is compiled using a concatenation of the sender text. In this example the various portions of sender text are simply combined or concatenated together.

In another example the electronic medical report is compiled using a combination of the sender text using a text composition large language model that outputs the electronic medical report in response to receiving the primary sender text as input. In this example, the various parts of the sender text are combined together using the text composition large language model. This may be useful in combining the sender text in a more readable fashion.

The text composition large language model may be implemented, for example using GPT-2, GPT-3, BERT, or LLaMA. The text composition large language model may be further trained by using fine-tuning as described above to provide the combined text. Data such as example sender texts with ground truth electronic medical reports may be used for the fine-tuning process.

In another example the electronic medical report is compiled by filling a text template with the sender text. In this example there is a text template which simply inserts the answer to the various questions and the location within the text template. This example may be beneficial because it may provide for a consistent means of providing the electronic medical report.

In another example the memory further comprises an audio-to-text conversion module that is configured to output text in response to receiving audio data comprising speech. Execution of the machine-executable instructions further causes the computational system to receive sender audio text using the sender user interface in response to sequentially present the one or more questions. The sender text is received by inputting the sender audio data into the audio-to-text conversion module. This example may be beneficial because it may provide a convenient dictation tool that aids a physician in responding to the electronic medical report in an efficient manner.

The audio-to-text conversion module may be implemented using commercially available audio-to-text conversion programs.

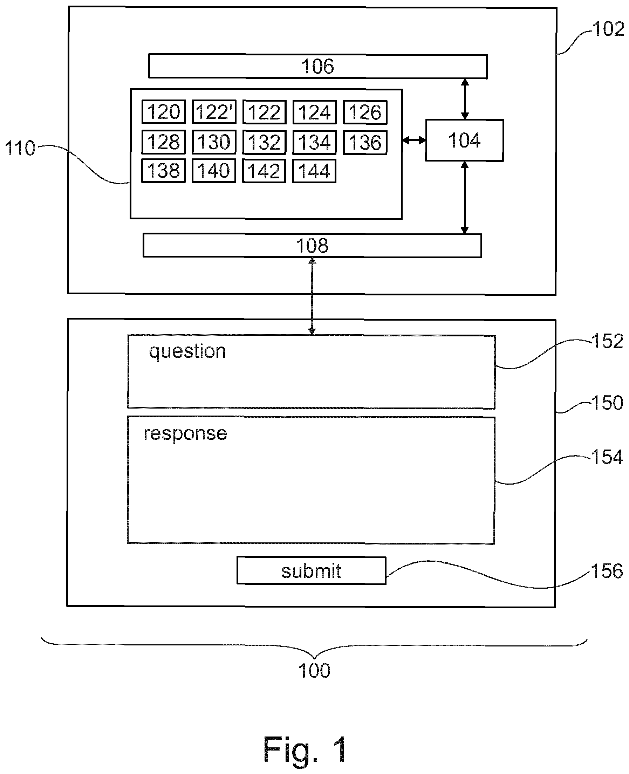

Fig. 1 illustrates an example of a medical system 100. In this example the medical system 100 comprises a computer 102. The computer 102 is intended to represent one or more computers or computer systems that are located at one or more locations. The computer 102 is shown as comprising a computational system 104. The computational system 104 is intended to represent one or more processor or computing systems that are located at one or more locations. The computational system 104 is shown as being in communication with an optional network or hardware interface 106. The network or hardware interface 106 may enable the computational system 104 to communicate with other computer systems and/or control other components of the medical system 100 if they are present. The computational system 104 is further shown as being in communication with an optional user interface 108 that may enable an operator or user of the medical system 100 to operate and control it. The computational system 104 is shown in further communication with a memory 110. The memory 110 is intended to represent different types of memory which are accessible to the computational system 104. In one example, the memory 110 is a non-transitory storage medium.

The memory 110 is shown as containing machine-executable instructions 120. The machine-executable instructions 120 may enable the computational system 104 to perform such tasks as controlling other components of the medical system 100, to perform basic text and/or numerical manipulations, and even to possibly reconstruct medical images. The memory 110 is further shown as containing receiver-specific metadata 122. This may be a collection of receiver-specific metadata 122 that may be useful for different users or targets. The memory 110 is further shown as containing receiver-specific metadata of a primary receiver 122'. This is receiver-specific metadata for a particular receiver. The memory 110 is further shown as containing a database of questions 124. These are questions which can be posed to a sender or someone who is compiling or writing an electronic medical report 132.

The memory 110 is further shown as containing a selection of a primary receiver 126. The primary receiver 126 is an individual or organization to which the electronic medical report will be sent. The memory 110 is further shown as containing one or more questions 128 that were retrieved from the database of questions 124 using the receiver-specific metadata of the primary receiver 122' and subject metadata 130. The memory 110 is further shown as also containing the subject metadata 130. The subject metadata 130 is data which is descriptive of a subject which is the topic of the electronic medical report 134. The memory 110 is further shown as also containing sender text 132 that was provided in response to posing the one or more questions 128. The memory 110 is further shown as also containing the electronic medical report 134 that was compiled from the sender text 132.

The memory 110 is further shown as also containing an optional audio-to-text conversion module 136. The memory 110 is also shown as also containing sender audio data 138. In some examples, instead of typing the sender text 132, the operator of the medical system 100 may dictate sender audio data 138 which is automatically converted to the sender text 132 by the audio-to-text conversion module 136.

The memory 110 is further shown as also containing an optional response checking large language model 140. The response checking large language model 140 is a large language model that has been configured to receive the sender text 132 and the one or more questions 128 as input and then to output auto complete text 142. The auto complete text 142 may for example be topics or information which was not present in the sender text 132 but would be desirable to be in the text because it is asked about in the one or more questions 128.

The memory 110 is further shown as also containing an optional text composition large language model 144. The text composition large language model is a large language model which has been configured to receive one or more portions of sender text 132 and then to compile them into the electronic medical report 134.

The medical system 100 is shown as optionally containing a sender user interface 150. In this example the sender user interface 150 displays at least one question display region 152, which is configured to display the one or more questions 128 and has a sender text input region 154, which is configured to receive the sender text 132. Once the sender text 132 has been entered, the operator may click the submit button 156 to send the sender text 132 to the computational system 104. In some examples, the audio-to-text conversion module 136 may be used to input text into the sender text input region 154.

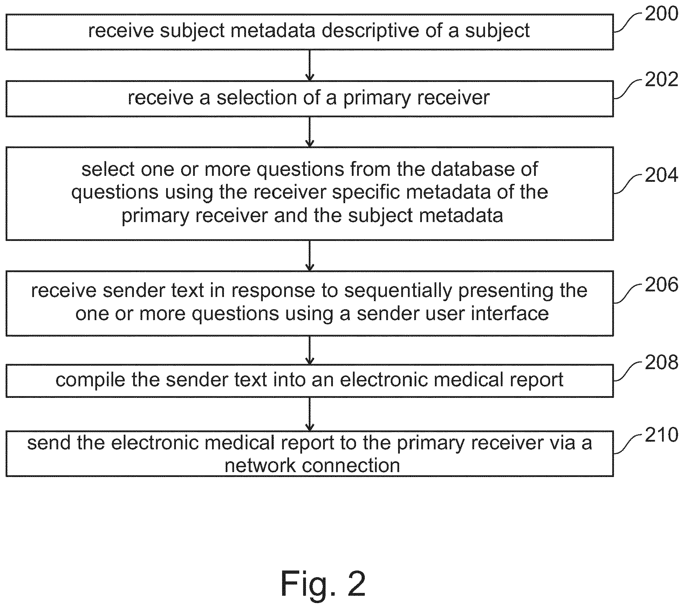

Fig. 2 shows a flowchart which illustrates a method of operating the medical system 100 of Fig. 1. First, in step 200, the subject metadata 130 is received. Next, in step 202, the selection of the primary receiver 126 is received. In step 204, the one or more questions 128 are selected or received from the database of questions 124 using the subject metadata 130 and the selection of the primary receiver 126. In step 206, the sender text 132 is received in response to sequentially presenting the one or more questions 128 using the sender user interface 150. In step 208, the electronic medical report 134 is compiled from the sender text 132. In step 210, the electronic medical report is sent to the primary receiver via a network connection. The network connection may for example be formed using the network interface 106.

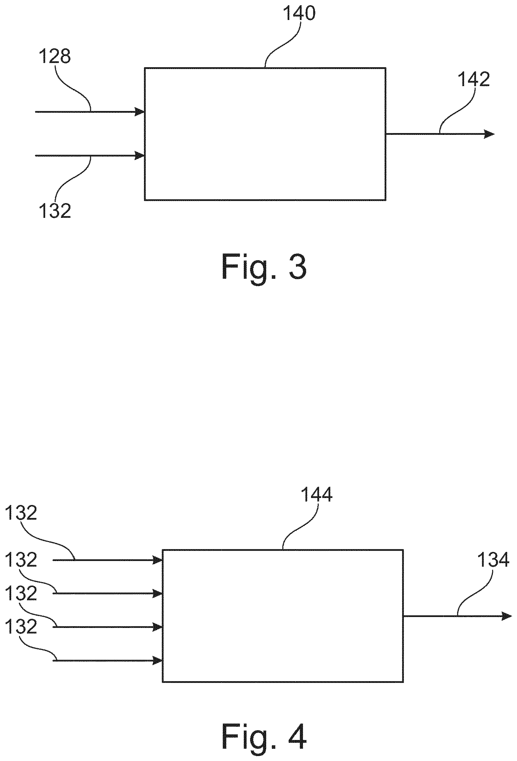

Fig. 3 illustrates an example of a response checking large language model 140. In this example, the response checking large language model 140 is configured to receive the one or more questions 128 and the sender text 132 as input. The response checking large language model 140 is then configured to output a sender message 142. In some examples this may be an auto complete text 142 that may be used as a suggested text to append or include in the sender text 132 to ensure that the one or more questions 128 are answered completely.

Fig. 4 illustrates an example of a text composition large language model 144. The text composition large language model 144 is a large language model that has been configured to receive one or more sender text 132. The group of sender text 132 is then combined by the text composition large language model 144 into the electronic medical report 134.

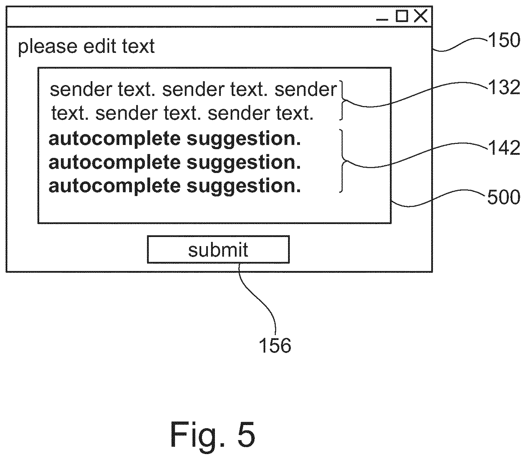

Fig. 5 illustrates a further example of a sender user interface 150. In this example the sender user interface 150 contains a text edit box 500 that is shown as containing sender text 132 along with the auto complete text 142 that may have been output by the response checking large language model 140. The user can then edit the sender text 132 as well as the auto complete suggestion 142. Once the text has been edited to satisfaction, the user may click the submit button 156 to submit the combined sender text and auto complete suggestion 142.

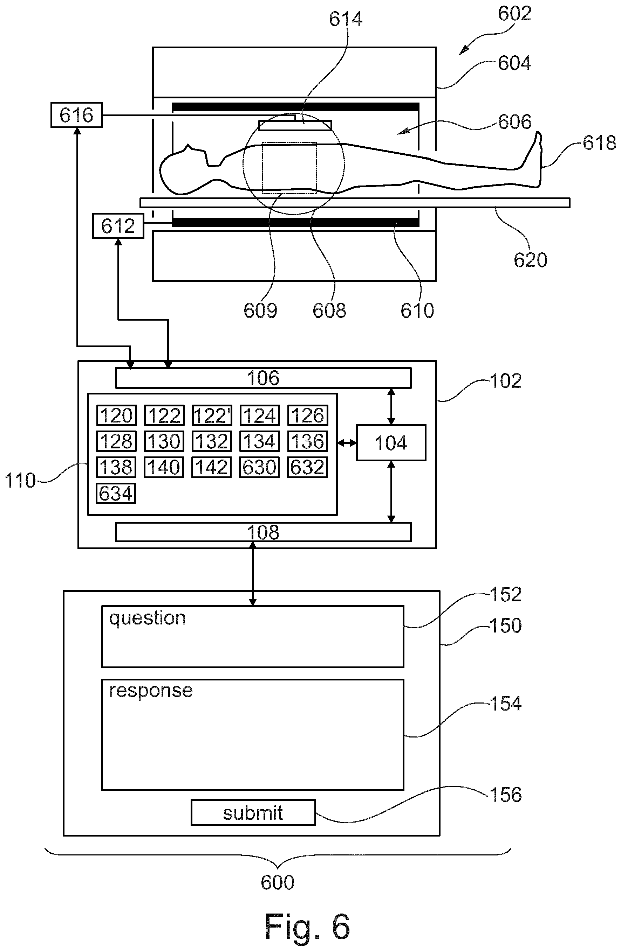

Fig. 6 illustrates a further example of a medical system 600. The medical system 600 in Fig. 6 is similar to the medical system 100 in Fig. 1 except that it additionally comprises a magnetic resonance imaging system 602.

The magnetic resonance imaging system 602 comprises a magnet 604. The magnet 604 is a superconducting cylindrical type magnet with a bore 606 through it. It is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject. The arrangement of the two sections area is similar to that of a Helmholtz coil. Open magnets are popular because the subject is less confined. Inside the cryostat of the cylindrical magnet there is a collection of superconducting coils.

Within the bore 606 of the cylindrical magnet 604 there is an imaging zone 608 where the magnetic field is strong and uniform enough to perform magnetic resonance imaging. A field of view 609 is shown within the imaging zone 608. The k-space data are acquired for the field of view 609. The region of interest could be identical with the field of view 609 or it could be a sub volume of the field of view 609. A subject 618 is shown as being supported by a subject support 620 such that at least a portion of the subject 618 is within the imaging zone 608 and the field of view 609.

Within the bore 606 of the magnet there is also a set of magnetic field gradient coils 610 which is used for the acquisition of measured k-space data to spatially encode magnetic spins within the imaging zone 608 of the magnet 604. The magnetic field gradient coils 610 are connected to a magnetic field gradient coil power supply 612. The magnetic field gradient coils 610 are intended to be representative. Typically, magnetic field gradient coils 610 contain three separate sets of coils for spatial encoding in three orthogonal spatial directions. A magnetic field gradient power supply supplies current to the magnetic field gradient coils. The current supplied to the magnetic field gradient coils 610 is controlled as a function of time and may be ramped or pulsed.

Adjacent to the imaging zone 608 is a radio frequency coil 614 for manipulating the orientations of magnetic spins within the imaging zone 608 and for receiving radio transmissions from spins also within the imaging zone 608. The radio frequency antenna may contain multiple coil elements. The radio frequency antenna may also be referred to as a channel or antenna. The radio frequency coil 614 is connected to a radio frequency transceiver 616. The radio frequency coil 614 and radio frequency transceiver 616 may be replaced by separate transmit and receive coils and a separate transmitter and receiver. It is understood that the radio frequency coil 614 and the radio frequency transceiver 616 are representative. The radio frequency coil 614 is intended to also represent a dedicated transmit antenna and a dedicated receive antenna. Likewise, the transceiver 616 may also represent a separate transmitter and receiver. The radio frequency coil 614 may also have multiple receive/transmit elements and the radio frequency transceiver 616 may have multiple receive/transmit channels. The transceiver 616 and the gradient controller 612 are shown as being connected to the hardware/network interface 106 of the computer system 102.

The memory 110 is shown as containing pulse sequence commands 630. The pulse sequence commands are commands or data, which may be converted into commands, to control the magnetic resonance imaging system 602 to acquire k-space data 632 according to a medical imaging protocol. The details of the pulse sequence commands 630 or the magnetic resonance imaging protocol may be used to select or at least partially select the one or more questions 128 from the database of questions 124. This may have the advantage of providing for questions 128, which are relevant to the particular pulse sequence commands 630 or its corresponding magnetic resonance imaging protocol. The memory 110 is further shown as containing a magnetic resonance image 634 and the k-space data 632. The pulse sequence commands 630 were used to control the magnetic resonance imaging system 602 to acquire the k-space data 632. The k-space data 632 is an example of medical image data. The magnetic resonance image 634 is an example of a medical image.

In some examples, the magnetic resonance image 634 may also be inserted to or included in the electronic medical report 134. The one or more questions 128, which are specific to the magnetic resonance image 634, may for example be used to annotate or provide specific information on the magnetic resonance image 634.

In Fig. 6 a specific example of the magnetic resonance imaging system 602 was used. This is however exemplary, and it may apply to other types of medical imaging systems such as: an ultrasound system, a computed tomography system, a tomographic medical imaging system, a positron emission tomography system, a single photon emission tomography system, a digital X-ray system, or digital fluoroscope.

Some examples may have one or more of the following features:
o the digital report (electronic medical report 134) may be structured in a way that a personalized (according to the individual preferences/needs of the receiver (using the receiver specific metadata 122, 122') list of questionnaires (one or more questions 128) can be followed. E.g. recipient X may always ask for specific information on diagnosis tools, and recipient Y may always ask for precise data numbers. Once knowing these facts from practice and/or data bases (where individual preferences are archived) the report is adapted to prevent time-consuming extra action to provide the specific information for recipient X and Y;
o the information (sender text 132) entered in the form may be compared to a template of possible and relevant questions posed by previous recipients to identify if any potentially requested information is missing;
o Based on the recipient's specific expertise (e.g., on disease, procedure, etc.) specific pieces of information can be queried and selected from relevant subject areas or domains and their properties and the relations between them can explicitly be shown;
o A measure for frequency of departure/divergence from a basic template [individual/discipline/department] can be determined and possibly be shown;
o Specific omissions can be identified and presented to the user as auto-completion suggestions.

Further examples are detailed below:
- Example: There may be an option to automatically flag and store a report/requisition that is automatically identified as containing omissions for later action by the recipient - e.g., when the recipient is less busy. Whether there is an omission or not could be decided based on the items that are usually looked at or requested by the colleagues that are addressed in the report, or by the set of colleagues the author usually cooperates with. Also, the role of the sender could be considered when relevant fields are omitted in the report (e.g., the relevant fields of a report might differ for a physician or a radiologist). The inventors also include the possibility to flag for review earlier. Often a report might only be reviewed shortly prior to seeing the patient, which might be too late to request extra information. If we flag it to the requestor in advance that info might be missing, then they can review and request with sufficient time before seeing the patient again. Especially important for patients outside the hospital / GP clinics.
- Example: Add information on the interaction between the information needs of the recipient (X, Y), and the patient case (A, B). For instance, when recipient X is presented with case type A, recipient X might request data, but when presented with case B might request medical background. This might be different for recipient B, who might request medical background when presented with case A, and other contextual variables when presented with case B.
- Example: Include the case where X requests a study / imaging to be done, but an unknown Z receives it and has to interpret it. Personalizing to X might not be sufficient, and instead X+Z is safer. This can often be the case in larger GP clinics, or in ICUs where staff shift change might happen between request and receipt. This may use a measure of whom receives the report.
- Example: Additionally, it comprises a module to determine communication scores calculated for automatically generated digital medical reports between pairs of stakeholders in an institution. These scores can include number of requests for clarification that is made for each report. Alternatively, the score can be based upon the time needed to inspect the report (maybe weighed for the number of measurements presented or for some other measure of overall case complexity). Such scores help to understand possible issues/inadequacies in the work of a person by various means, e.g.:
   o for a pair of stakeholders, A and B, the communication scores can be normalized by average scores for communication involving either A or B;
   o Average communication scores for a person can be compared against institutional or department average to identify outliers;
   o Effective interdisciplinary communication can be characterized through comparing averaged scores for communication between departments;
   o for both interdisciplinary or interpersonal communication, effectiveness may be further granularized by analyzing scores for study types, indications, patient demographic factors etc.
- Example: Metrics on communication between pairs of stakeholders can be reused the next time they interact with each other (or with others), in the form of 'pop-ups' while making a new entry, showing for example "previous time you contacted clinician X they missed information Y, please consider that when writing your new message". If this is perceived as obtrusive, a policy can be in place where the direct/non-anonymized recipient can flag the interlocutor so that such a notification appears for their subsequent interactions.

It is understood that one or more of the aforementioned embodiments or examples of the invention may be combined as long as the combined embodiments or examples are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a `human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

K-space data is defined herein as being the recorded measurements of radio frequency signals emitted by atomic spins using the antenna of a Magnetic resonance apparatus during a magnetic resonance imaging scan.

A Magnetic Resonance Imaging (MRI) image or MR image is defined herein as being the reconstructed two- or three-dimensional visualization of anatomic data contained within the k-space data. A magnetic resonance image is an example of medical imaging data.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: computational system
- 106: network / hardware interface
- 108: user interface
- 110: memory
- 120: machine executable instructions
- 122: receiver specific metadata
- 122': receiver specific metadata of primary receiver
- 124: database of questions
- 126: selection of a primary receiver
- 128: one or more questions
- 130: subject metadata
- 132: sender text
- 134: electronic medical report
- 136: audio-to-text conversion module
- 138: sender audio data
- 140: response checking large language module (LLM)
- 142: auto complete text (sender message)
- 144: text composition large language model
- 150: sender user interface
- 152: at least one questions display region
- 154: sender text input region
- 156: submit button
- 200: receive subject metadata descriptive of a subject
- 202: receive a selection of a primary receiver
- 204: select one or more questions from the database of questions using the receiver specific metadata of the primary receiver and the subject metadata
- 206: receive sender text in response to sequentially presenting the one or more questions using a sender user interface
- 208: compile the sender text into an electronic medical report
- 210: send the electronic medical report to the primary receiver via a network connection

- 500: text edit box
- 600: medical system
- 602: magnetic resonance imaging system
- 604: magnet
- 606: bore of magnet
- 608: imaging zone
- 609: field of view
- 610: magnetic field gradient coils
- 612: magnetic field gradient coil power supply
- 614: radio frequency coil
- 616: transceiver
- 618: subject
- 620: subject support
- 630: pulse sequence commands
- 632: k-space data (medical image data)
- 634: magnetic resonance image

## Claims

1. A medical system (100, 600) comprising:
- a memory (110) storing machine executable instructions (120), receiver specific metadata (122, 122'), and database of questions (124); wherein the receiver specific metadata comprises pointers to at least a portion of the database of questions;
- a computational system (104), wherein execution of the machine executable instructions causes the computational system to:
- receive (200) subject metadata (130) descriptive of a subject (618);
- receive (202) a selection (126) of a primary receiver;
- select (204) one or more questions (128) from the database of questions using the receiver specific metadata (122') of the primary receiver and the subject metadata;
- receive (206) sender text (132) in response to sequentially presenting the one or more questions using a sender user interface;
- compile (208) the sender text into an electronic medical report (134); and
- send (210) the electronic medical report to the primary receiver via a network connection.

2. The medical system of claim 1, wherein the memory further comprises a response checking module (140), wherein the response checking module is configured to output an answer rating (142) in response to receiving the one or more questions and the sender text as input, wherein execution of the machine executable instructions further causes the computational system to:
- receive the answer rating in response to inputting the one or more questions and the sender text as input;
- present a sender message using the sender user interface if the answer rating does not meet a predetermined criterion.

3. The medical system of claim 2, wherein the response checking module is implemented as any one of the following: a text template, a response checking large language model (140), and combinations thereof.

4. The medical system of claim 2 or 3, wherein the sender message comprises any one of the following:
- an autocomplete suggestion (142);
- an incomplete text warning;
- a prompt to enter additional text; and
- combinations thereof.

5. The medical system of claim 2, 3, or 4, wherein execution of the machine executable instructions further causes the computational system to:
- store the sender text and the sender message in the memory; and
- trigger the presentation of the sender message using the sender user interface if a predetermined sender activity criterion is met or after a predetermined delay.

6. The medical system of any one of the preceding claims, wherein execution of the machine executable instructions further causes the computational system to:
- receive a selection of an alternative receiver; and
- filter and/or reformat the sender text using the receiver specific metadata of the alternative receiver, wherein the electronic medical report is compiled from the filtered and/or reformatted sender text.

7. The medical system of any one of the preceding claims, wherein execution of the machine executable instructions comprises inserting medical image data (634) descriptive of the subject into the electronic medical report, wherein at least a portion of the subject metadata is descriptive of the medical image data.

8. The medical system of claim 7, wherein at least a portion of the subject text is inserted into the electronic medical report as an annotation of the medical image data.

9. The medical system of claim 7 or 8, wherein the medical system further comprises a medical imaging system (602) configured for acquiring the medical imaging data descriptive of the subject, wherein the memory further stores configuration commands configured for controlling the medical imaging system to acquire the medical imaging data, wherein execution of the machine executable instructions further causes the computational system to control the medical imaging system with the configuration commands to acquire the medical imaging data from the subject, wherein the one or more questions are at least partially selected from the database of questions using the configuration commands.

10. The medical system of any one of the preceding claims, wherein execution of the machine executable instructions further causes the computational system to:
- receive feedback data from the primary receiver in response to sending the electronic medical report to the primary receiver via a network connection; and any one of the following:
- modify the receiver specific metadata and/or the database of questions using the feedback data;
- control popup messages on the sender user interface during subsequent reception of the sender text by the sender user interface; and
- combinations thereof.

11. The medical system of claim 10, wherein the feedback data comprises any one of the following: a communication score ranking the electronic medical report, at least one request for clarification, a number of requests for clarification, a measured time to study the electronic medical report, and combinations thereof.

12. The medical system of any one of the preceding claims, wherein the electronic medical report is compiled using any one of the following:
- a concatenation of the sender text;
- a combination of the sender text using a text composition large language model (144) that outputs the electronic medical report in response to receiving the primary sender text as input;
- filling a text template with the sender text; and
- combinations thereof.

13. The medical system of any one of the preceding claims, wherein the memory further comprises an audio-to-text conversion module configured to output text in response to receiving audio data comprising speech, wherein execution of the machine executable instructions further causes the computational system to receive sender audio data using the sender user interface in response to sequentially presenting the one or more questions, wherein the sender text is received by inputting the sender audio data into the audio-to-text conversion module.

14. A method of generating an electronic medical report (134), wherein the method comprises:
- receiving (200) subject metadata (130) descriptive of a subject (618);
- receiving (202) a selection of a primary receiver (126);
- selecting (204) one or more questions (128) from the database of questions using the receiver specific metadata (122') of the primary receiver and the subject metadata;
- receiving (206) sender text (132) in response to sequentially presenting the one or more questions using a sender user interface;
- compiling (208) the electronic medical report from the sender text into an electronic medical report (134); and
- sending (210) the electronic medical report to the primary receiver via a network connection.

15. A computer program comprising machine executable instructions (120), wherein execution of the machine executable instructions causes a computational system to:
- receive (200) subject metadata (130) descriptive of a subject (618);
- receive (202) a selection of a primary receiver (126);
- select (204) one or more questions (128) from a database of questions using receiver specific metadata (122') of a primary receiver and the subject metadata;
- receive (206) sender text (132) in response to sequentially presenting the one or more questions using a sender user interface;
- compile (208) the electronic medical report from the sender text into an electronic medical report; and
- send (210) the electronic medical report to the primary receiver via a network connection.
